(19) Europäisches Patentamt
European Patent Office
Office européen des brevets

(11) Veröffentlichungsnummer: **0 254 091**
**B1**

(12) **EUROPÄISCHE PATENTSCHRIFT**

(45) Veröffentlichungstag der Patentschrift: **07.02.90**

(51) Int. Cl.[4]: **C07C 69/145**, C07C 67/293

(21) Anmeldenummer: **87109500.6**

(22) Anmeldetag: **02.07.87**

# (54) Verfahren zur Herstellung von 1-Acetoxy-2,4-hexadien.

(30) Priorität: **19.07.86 DE 3624438**

(43) Veröffentlichungstag der Anmeldung:
**27.01.88 Patentblatt 88/4**

(45) Bekanntmachung des Hinweises auf die Patenterteilung:
**07.02.90 Patentblatt 90/6**

(84) Benannte Vertragsstaaten:
**CH DE FR GB LI NL**

(73) Patentinhaber: **BASF Aktiengesellschaft, Carl-Bosch-Strasse 38, D-6700 Ludwigshafen(DE)**

(72) Erfinder: **Barth, Wolfgang, Dr., Berthold-Schwarz-Strasse 15, D-6700 Ludwigshafen(DE)**
Erfinder: **Pape, Frank-Friedrich, Dr., Berner Weg 34, D-6700 Ludwigshafen(DE)**
Erfinder: **Janitschke, Lothar, Dr., Wormser Gasse 9 a, D-6711 Kleinniedesheim(DE)**

(56) Entgegenhaltungen:
**WO-A-84/00954**
**US-A-3 755 451**

**CHEMICAL ABSTRACTS, Band 41, Nr. 14, 20. Juli 1947, Spalte 4507, Columbus, Ohio, US; E.R.H. JONES "New unsaturated alcohols and their esters and ethers"**
**CHEMICAL ABSTRACTS, Band 43, Nr. 1, 10. Januar 1949, Spalte 112, Columbus, Ohio, US; I.N. NAZAROV "LXI. Rearrangements of 1,3-diene systems. 3. Reversible isomerization of 1,3-hexadien-5-ol into 2,4-hexadien-1-ol (sorbic alcohol) and the rearrangement of the corresponding chlorides in an exchange reaction" FIZ.-KHIM. OSN. SOZDANIYA ZHAROPROCHN. ME 000**
**CHEMICAL ABSTRACTS; Band 50, Nr. 16, 25. August 1956, Spalte 11227, Columbus, Ohio, US; C.**

(56) Entgegenhaltungen: (Fortsetzung)
**PREVOST "A generalization on the allytic transposition"**
**CHEMICAL ABSTRACTS, Band 73, Nr. 18, 2. November 1970, Seite 76, Zusammenfassungsnr. 89245m, Columbus, Ohio, US; C.L. MILLIGAN "Hexadienol and hexadienal derivatives useful as reactive diluents in drying oil coatings"**
**JOURNAL OF THE CHEMICAL SOCIETY, CHEMICAL COMMUNICATIONS, Nr. 20, 1981, Seite 1030-1031; B.T. GOLDING et al.: "PdII catalysed isomensations of 3-acetoxy-1,4-dienes to 1-acetoxy-2,4-dienes: Stereochemical and propanative aspects"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 254 091 B1

ACTORUM AG

## Beschreibung

Die Erfindung betrifft ein Verfahren zur Herstellung von 1-Acetoxy-2,4-hexadien (Sorbylacetat) durch Erhitzen des z.B. aus Crotonaldehyd und Vinylmagnesiumchlorid leicht zugänglichen 1,4-Hexadien-3-ols (W. Oppolzer, R.L. Snowden, D.P. Simmons, Helv. Chim. Acta 64 (1981), 2002) in einer Mischung aus Eisessig und Alkaliacetat.

Sorbylacetat ist ein gesuchtes Zwischenprodukt u.a. für die Herstellung von Blätteralkohol oder des Sexualpheromons des Apfelwicklers (Laspeyresia pomonella) "Codlemon" (vgl. C.A. Henrick, Tetrahedron 33 (1977), 1845; EP 3708). Sorbylacetat per se ist ein Naturstoff. Es wurde im Duftsekret verschiedener Insekten gefunden (Oncopeltus fasciatus, Experientia 29 (1973), 532; Leptoglossus, Z.f. Naturforschung C, 1985, 142).

Es fehlt daher nicht an Verfahren, ausreichende Mengen an Sorbylacetat zu synthetisieren. Bevorzugte Ausgangsmaterialien sind Sorbinsäureester oder -anhydride. Die Ausbeuten sind zufriedenstellend (ca. 60 %, US 4 526 993, DuPont, 02.07.1985), jedoch verteuert der Einsatz von Natriumborhydrid das über 2,4-Hexadienol gewonnene Sorbylacetat erheblich.

Weiterhin wird zur Herstellung des Sorbylacetats ein weiterer Reaktionsschritt benötigt, nämlich die Acylierung von 2,4-Hexandienol mit z.B. Acetanhydrid (in 90 %iger Ausbeute, vgl. D. Samain, C. Descoins, Synthesis 1978, 388). Eine Alternative bietet Sorbylaldehyd, der sich gut mit Natriumborhydrid reduzieren läßt. 2,4-Hexadienal (sorbylaldehyd) wird aus Acetaldehyd und Crotonaldehyd durch einfache Aldolkondensation hergestellt. 2,4-Hexandienal wurde jedoch mit nur 17 %iger Ausbeute isoliert, anschließende Reduktion ergab 2,4-Hexadienol mit 86 %iger Ausbeute (A. Furuhata, S.E. Ryo, A. Fujita, K. Kogami, VIIIth International Congress of Essential Oils, Oct. 1980, Technical Datas, S. 554).

Es bestand daher die Aufgabe ein Verfahren zur Herstellung von Sorbylacetat vorzuschlagen, das technisch einfach und ausgehend von billigen Einsatzmaterialien in guten Ausbeuten durchzuführen ist. Dazu wurde schon versucht, Sorbylacetat aus 1,4-Hexadien-3-ol (1) herzustellen. 1,4-Hexadien-3-ol kann technisch einfach durch Vinylmagnesiumchloridaddition an Crotonaldehyd in 80 %iger Ausbeute hergestellt werden (vgl. W. Oppolzer, l.c.). Acylierung mit Acetanhydrid liefert 1,4-Hexadienyl-3-acetat (2) (vgl. A. Henrick, Tetrahedron 33 (1977), 1845). Das Acetat 2 läßt sich mit $Pd^{II}$-oder $Pt^{IV}$-Salzen zu 1,3-Hexadienyl-5-acetat (3), mit $Pd^0$-Komplexen zu Sorbylacetat (4) isomerisieren (vgl. B.T. Golding. C. Pierpoint, R. Aneja, Chem. Commun. 1981, 1030).

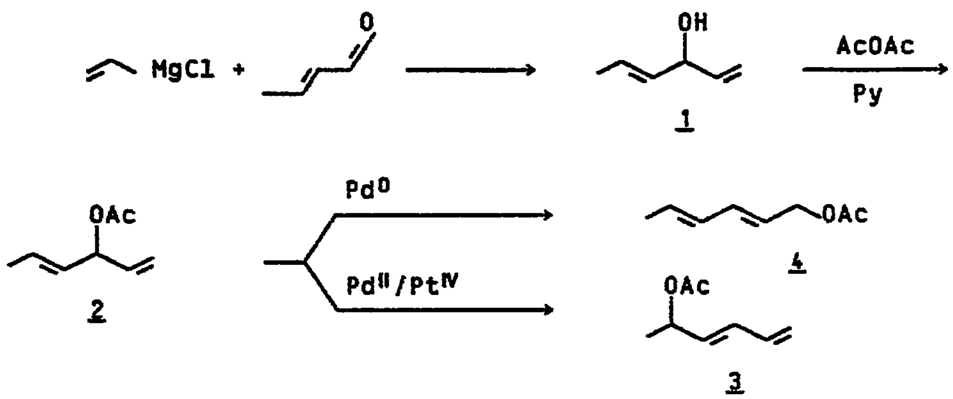

1
2
4
3

Die $Pd^0$ katalysierte Umlagerung von 2 zu 4 verläuft mit 50 bis 60 %iger Ausbeute. Der Einsatz von Tetrakis-(triphenylphosphin)-palladium (d.i. ein gängiger $Pd^0$-Komplex) verteuert jedoch diesen Syntheseweg in nicht akzeptabler Weise.

Es wurde nun überraschenderweise gefunden, daß sich 1,4-Hexadien-3-ol mit Eisesig und Alkaliacetat ohne die Notwendigkeit der Verwendung von Palladium in ein Gemisch aus 1,3-Hexadienyl-5-acetat (3) und 2,4-Hexadienyl-1-acetat (4) überführen läßt. Bei einer Reaktionstemperatur von 25 bis 50°C liegt das Isomerenverhältnis 3 : 4 auf Seiten des unerwünschten 3. Bei Erhöhung der Reaktionstemperatur verschiebt sich jedoch das Isomerenverhältnis weit zugunsten des gewünschten 4. Bei einer Reaktionstemperatur von 120°C erhält man z.B. ein 1:2-Gemisch der Isomeren 3 und 4 in 60 bis 70 %iger Gesamtausbeute. 3 und 4 lassen sich destillativ trennen, 3 kann erneut mit dem erfindungsgemäßen Verfahren in ein 2:1-Gemisch aus 4 und 3merisiert werden. Die Gesamtausbeute an 2,4-Hexadienyl-1-acetat, bezogen auf Crotonaldehyd, beträgt ca. 46 %.

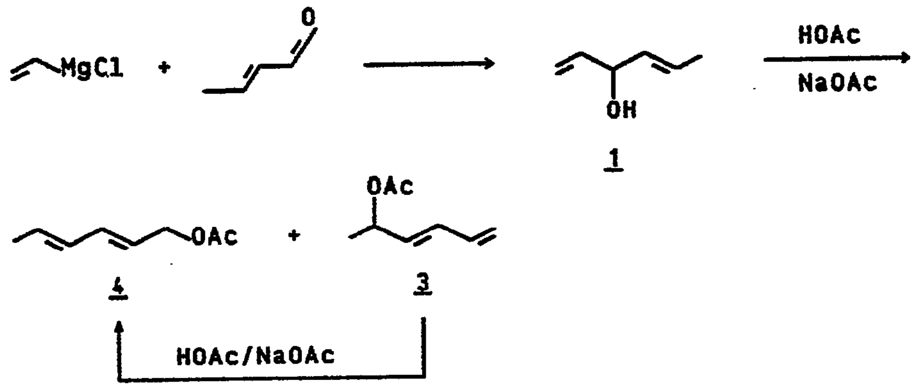


1 4 3

Nach einer gaschromatographischen Analyse (50 m WG-11, 160°C) besteht das so hergestellte 2,4-Hexadienyl-1-acetat zu 89 % aus dem all-E-Isomeren, zu 9,3 % aus dem 2-E,4-Z- und zu 1,5 % aus dem 2-Z, 4-E-Isomeren.

Die erfindungsgemäße Acetylierung und Isomerisierung wird in der Regel bei Temperaturen von 50 bis 130°C, vorzugsweise 80 bis 125°C und insbesondere bei Siedetemperatur des Eisessig/Alkaliacetatgemisches durchgeführt.

Das dem Eisessig zuzusetzende Alkali wird im Gewichtsverhältnis Eisessig zu Natriumacetat von 1:1 bis 10:1, vorzugsweise ungefähr 4:1 angewandt. Als Alkaliacetat kommen vor allem Kaliumacetat und insbesondere Natriumacetat in Betracht. Jedoch sind auch andere Alkaliacetate und auch Ammoniumacetat verwendbar.

Das Eisessig/Alkaliacetat-Gemisch soll praktisch wasserfrei sein, doch können bis zu 5 Gew.% Wasser toleriert werden.

Die Menge des Eisessig/Alkaliacetat-Gemisches wird in der Regel im 5 bis 20, vorzugsweise 9 bis 12fachen Gewichtsüberschuß, bezogen auf das 1,4-Hexadien-3-ol, angewandt.

Zur Aufarbeitung verdünnt man die Reaktionsmischung zweckmäßig mit Wasser und extrahiert die acetylierten Produkte. Aus dem Extrakt kann durch fraktionierte Destillation Sorbylacetat gewonnen werden. Das unerwünschte Isomere 5-Acetoxy-1,3-hexadien (3) wird durch Erwärmen in Eisessig/Alkaliacetat in ein Gemisch aus Sorbylacetat (4) und unverändertem 5-Acetoxy-1,3-hexadien (3) isomerisiert und daraus Sorbylacetat isoliert oder in die Reaktionsmischung der Acetylierung/Isomerisierung von 1,4-Hexadien-3-ol zurückgeführt.

Der oben beschriebene Weg ermöglicht also ein Verfahren zur Herstellung von Sorbylacetat, das von billigen Einsatzstoffen ausgeht (Crotonaldehyd, Vinylmagnesiumchlorid, Eisessig, Natriumacetat), sich in zwei Stufen leicht durchführen läßt und mit guter Gesamtausbeute verläuft.

Beispiel 1

(a) 1,4-Hexadien-3-ol (1)

Zu 3 l einer 1,5 molaren Vinylmagnesiumchloridlösung in Tetrahydrofuran wurden bei Raumtemperatur 280 g (4,0 Mol) Crotonaldehyd während 2 Stunden zugetropft. Die Reaktionsmischung wurde über Nacht bei Raumtemperatur gerührt und dann mit 450 ml Wasser versetzt. Vom ausgefallenen Magnesiumhydroxid wurde abgesaugt, der Filterkuchen mit Tetrahydrofuran gewaschen und die vereinigten organischen Phasen wurden eingeengt. Dach Destillation des Rückstandes (73°C/60 Torr) erhielt man 328 g (3,35 Mol, 84 %) 1,4-Hexadien-3-ol.

(b) 1-Acetoxy-2,4-hexadien (4) Sorbylacetat) und 5-Acetoxy-1,3-hexadien (3)

262 g (2,5 Mol) 1,4-Hexadien-3-ol wurden während 5 Stunden in einer Mischung aus 2,5 l Eisessig und 600 g Natriumacetat am Rückfluß zum Sieden erhitzt (123°C). Nach Abkühlung auf 80°C versetzte man mit 1 l Wasser und fügte 500 ml n-Hexan zu. Nach Trennung wurde die wäßrige Phase viermal mit je 750 ml Diethylether extrahiert (Extraktion mit Hexan führt zu Ausbeuteverminderung). Die vereinigten organischen Phasen wurden mit 10 %iger Natronlauge bis zur Neutralität gewaschen. Das Lösungsmittel wurde abdestilliert und der Rückstand über eine Claissenbrücke destilliert.

Man erhielt 258 g eines Gemisches, das laut gaschromatographischer Analyse (2m SE 30, 100°C) neben 2,1 % Ausgangsprodukt 34,1 % 3 und 62,1 % 4 enthält.

Ausbeute 4 und 3: 248 g (1,77 Mol, 71 %)
Ausbeute 4: 160 g (1,14 Mol, 46 %)

1340 g eines Gemisches aus 6 Reaktionsansätzen, die alle 3 und 4 im Verhältnis von ca. 34:63 enthielten, wurden über eine 1m Silberkolonne, mit Glasringen gefüllt, destilliert (Rücklaufverhältnis 30:1). Es wurden insgesamt 9 Fraktionen abgetrennt, die sich folgendermaßen zusammenfassen lassen:

| | | Vorlauf | 4 | 3 | |
|---|---|---|---|---|---|
| 1. | 32 g | 46% | 0,5% | 54,0% | 46– 68°C/24 Torr |
| 2. | 318 g | 2% | 3,0% | 95,0% | 68– 71°C/24 Torr |
| 3. | 156 g | – | 44,0% | 53,0% | 71– 90°C/24 Torr |
| 4. | 733 g | – | 98,0% | 1,5% | 98°C/26 Torr |

Eine genauere gaschromatographische Untersuchung (50m WG-11, 160°C) zeigte, daß das so hergestellte Sorbylacetat aus einem 9,3:89,2:1,5-Isomerengemisch besteht. Eine destillative Trennung dieser Isomere ist nicht möglich.

Ein $^{13}C$-NMR-Spektrum dieser Mischung zeigte ein 80:12:8-Gemisch der 2E,4E-2E,4Z- und 2Z,4E-Isomere.

Beispiel 2

Isomerisierung von 5-Acetoxy-1,3-hexadien (3) in 1-Acetoxy-2,4-hexadien (4)

412 g eines Gemisches aus 81 % 3 und 17,2 % 4 wurden während 5 Stunden in einer Lösung von 820 g Natriumacetat in 3 l eisessig am Rückfluß zum Sieden erhitzt. Nach Abkühlung wurde mit 1,5 l Wasser versetzt, fünfmal mit je 800 ml n-Hexan extrahiert, die vereinigten organischen Phasen wurden mit 10 %iger Natronlauge gewaschen und das Lösungsmittel wurde im Rotationsverdampfer abgezogen.

Nach Destillation des Rückstandes über eine Claissenbrücke erhielt man 285 g (69 %) einer 2,1:1-Mischung aus 4 und 3.

Vergleichsbeispiel 1

Isomerisierung von 3-Acetoxy-1,4-hexadien (2) mit $PtCl_4$

50 g 94 %iges 2 (0,34 Mol) wurden mit 0,3 ml Acetonitril (5,7 mMol) und 0,1 g (0,3 mMol) Platin-IV-chlorid auf 50°C erwärmt (Erwärmung auf 100°C führte zu Polymerisation). Ein nach 8stündiger Reaktionszeit aufgenommenes Gaschromatogramm (2m SE 30, 100°C) zeigte Sorbylacetat (4) und 5-Acetoxy-hexa-1,3-dien (3) im Verhältnis 7:93. Nach Destillation über eine Claissenbrücke (65-71°C/20 Torr) erhielt man 39 g (78 %) eines Gemisches, das laut gaschromatographischer Analyse (2m SE 30, 100°C) aus 3,7 % 2, 90,8 % 3 und 4,0 % 4 bestand.

Vergleichsbeispiel 2

Isomerisierung von 3-Acetoxy-1,4-hexadien (2) mit $(PPh_3)_4Pd$

Eine Lösung von 20 g (0,14 Mol) 2 und 1,0 g (0,86 mMol, 0,6 Mol%) Tetrakis(triphenylphosphin)-palladium in 80 ml Toluol wurde unter Luft- und Lichtausschluß 2 Tage bei 40°C gerührt. Zur Entfernung des Katalysators (wichtig, da sonst HOAc-Eliminierung) wurde nach Abziehen des Toluols im Rotationsverdampfer die Reaktionsmischung mit Ether durch eine kurze $SiO_2$-Säule gefiltert. Der Ether wurde im Rotationsverdampfer abgezogen und der Rückstand über eine Claissenbrücke destilliert.

Man erhielt 10 g (50 %) eines Gemisches aus 5,1 % 3 und 94,8 % 4.

Das so gewonnene Sorbylacetat 4 besteht laut gaschromatographischer Analyse (50m WG-11, 130°C) und $^{13}C$-NMR-Spektrum zu 90 % aus 1-Acetoxy-hexa-2-E,4-E-dien und zu 10 % aus 1-Acetoxy-hexa-2-E,4-Z-dien. Beide Isomere lassen sich destillativ nicht trennen.

**Patentansprüche**

1. Verfahren zur Herstellung von 1-Acetoxy-2,4-hexadien, dadurch gekennzeichnet, daß man 1,4-Hexadien-3-ol in einer Mischung aus Eisessig und Alkaliacetat auf Temperaturen über 50°C erhitzt.

2. Verfahren nach Anspruch 1, dadurch gekennzeichnet, daß man 1,4-Hexadien-3-ol in einer Mischung aus Eisessig und Natriumacetat zum Sieden erhitzt.

3. Verfahren nach Anspruch 1, dadurch gekennzeichnet, daß man das als Nebenprodukte gebildete 5-Acetoxy-1,3-hexadien durch Erhitzen in einer Mischung aus Eisessig und Alkaliacetat auf Temperaturen über 80°C isomerisiert und das gewünschte 1-Acetoxy-2,4-hexadien abtrennt oder das 5-Acetoxy-1,3-hexadien in die Reaktionsmischung der Umsetzung von 1,4-Hexadien-3-ol zurückführt.

## Claims

1. A process for the preparation of 1-acetoxy-2,4 hexadiene, wherein 1,4-hexadien-3-ol in a mixture of glacial acetic acid and an alkali metal acetate is heated to above 50°c.

2. A process as claimed in claim 1, wherein 1, 4-hexadien-3-ol in a mixture of glacial acetic acid and sodium acetate is heated to the boil.

3. A process as claimed in claim 1, wherein the 5-acetoxy-1,3-hexadiene formed as a byproduct is isomerized by heating in a mixture of glacial acetic acid and an alkali metal acetate to above 80°c and the desired 1-acetoxy-2,4-hexadiene is separated off, or the 5-acetoxy-1,3-hexadiene is recycled to the reaction mixture for the reaction of 1,4-hexadien-3-ol.

## Revendications

1. Procédé de préparation de 1-acétoxy-2, 4-hexadiène, caractérisé par le fait que l'on chauffe, à des températures supérieures à 50°C, du 1,4-hexadien-3-ole dans un mélange d'acide acétique et d'acétate alcalin.

2. Procédé selon la revendication 1, caractérisé par le fait que l'on chauffe à l'ébullition du 1,4-hexadien-3-ole dans un mélange d'acide acétique et d'acétate de sodium.

3. Procédé selon la revendication 1, caractérisé par le fait que l'on isomérise le 5-acétoxy-1,3-hexadiène, formé comme sous-produit, par chauffage dans un mélange d'acide acétique et d'acétate alcalin, à des températures supérieures à 80°C, et on sépare le 1-acétoxy-2,4-hexadiène souhaité, ou bien on renvoie le 5-acétoxy-1,3-hexadiène dans le mélange de la réaction de 1,4-hexadien-3-ole.